# EUROPEAN PATENT APPLICATION

(11) **EP 2 181 705 A1**
(43) Date of publication of application: **05.05.2010**
(21) Application number: 08291018.3
(22) Date of filing: 31.10.2008
(51) Int. Cl.: A61K 9/16, A61K 9/20, A61K 31/64

(54) **Sustained-release formulation of gliclazide**

(71) Applicant: Disphar International B.V., 7255 PZ Hengelo (NL)
(72) Inventor: Wit, Johannes Bernardus, Maria, 3742 ZD Baarn (NL); Doshi, Hiteshkumar Anilkant, Mulund (W), Mumbai 400 080 (IN)
(74) Representative: Hirsch & Associés

(57) **Abstract**

The present invention relates to a sugar-free sustained-release gliclazide formulation comprising an intragranular part and an extragranular part, wherein the intragranular part comprises gliclazide or a salt thereof and the extragranular part comprises a cellulose-derived polymer, said intragranular part being binder-free.

The sustained-release formulation of the invention preferably consists essentially of:
- gliclazide or a salt thereof;
- a diluent
- an extragranular cellulose-derived polymer;
- a lubricant and/or glidant.

The present invention also relates to a process for making the same.

## Description

### TECHNICAL FIELD

The present invention relates to sustained-release formulations of gliclazide or salts thereof and processes for their preparation.

### TECHNICAL BACKGROUND

Gliclazide (see US 4,056,623) is an antidiabetic drug from the sulfonylurea class that has been widely administered orally in the form of tablets. The usual average prescription regimen is two tablets per day in two administrations, but may vary from 1 to 4 slow release tablets (30 mg) per day in several administrations depending upon the severity of the diabetes.

Sustained-release formulations are particular pharmaceutical compositions which have the ability to maintain a desired blood level of a medicament over an extended period of time by minimizing the peak-trough variations in plasma concentrations. They may also increase patient compliance by reducing the number of administrations necessary to achieve a desired therapeutic effect. It is therefore desirable to provide sulfonylurea compositions (in particular gliclazide compositions) as sustained-release formulations having improved dissolution properties and extended release profiles.

However, since sulfonylurea compounds are relatively insoluble, they are difficult to incorporate into sustained-release formulations. It is often difficult to predict whether a particular sustained-release formulation will provide the desired release profile for a relatively insoluble compound.

EP 0468392 describes a process for preparing pharmaceutical compositions which allow the active substance to be released very rapidly into an aqueous medium. A hydrophilic polymer or a cyclodextrin is used to this effect.

US 4,696,815 relates to sulfonylurea formulations enabling a rapid release of the active substance. Polyvinylpyrrolidone is used as an additive.

CA 2173366 is directed to sulfonylurea compositions said to release the active substance rapidly or in a controlled manner. Polyols and sugar alcohols are used as additives in these compositions.

WO 99/18932 is directed to sulfonylurea (in particular glipizide) controlled-release formulations. The document teaches to include a gelling agent in the formulations. A mixture of xanthan gum, locust bean gum and dextrose is preferred.

In US 6,475,521 and US 6,524,618, controlled release formulations of metformin are disclosed. An antidiabetic agent may be provided in the formulations as well.

WO 00/18373 describes a controlled-release formulation of gliclazide, wherein use is made of the association of a cellulose-derived polymer and glucose syrup (notably maltodextrin). Diamicron® MR, commercialized by Servier, is a gliclazide formulation of this type. It comprises maltodextrin and hypromellose.

WO 2005/009412 is directed to formulations adapted to various combinations of drugs. In particular, the document teaches a specific formulation of metformin and gliclazide together with a cellulose-derived polymer and polyvinylpyrrolidone as a binder.

WO 2006/123213 is directed to modified release formulations comprising gliclazide, a controlled-release polymer (in particular a cellulose-derived polymer) and a binder. Polyvinylpyrrolidone is a preferred binder. The formulations described in the document are said to be bioequivalent with Diamicron® MR.

WO 2006/061697 is directed to sustained release compositions comprising sulfonylurea, a polymer, a disaccharide and/or a monosaccharide. Specific examples are provided of compositions comprising *inter alia* gliclazide, hydroxypropylmethylcellulose and lactose or dextrose.

WO 2008/062470 is directed to a pharmaceutical dosage form of gliclazide comprising one or more release controlling polymers, anhydrous calcium hydrogen phosphate and without saccharide component.

A problem encountered with most prior art sustained-release formulations of gliclazide is that it is generally undesirable to use sugar based compounds as excipients in the preparation of formulations to be administered for the treatment of diabetes. The use of such excipients may increase the blood sugar levels in diabetic patients.

Sustained-release formulations of gliclazide without any sugar compound have also been disclosed in the prior art. However, some of them provide the association of at least two classes of compounds, for example a controlled-release polymer and a binder (see WO 2006/123213). Besides, in all instances, the intragranular part of these formulations comprises one or more ingredients in addition to the active ingredient and a diluent, such as a release-controlling polymer or a binder, which may be detrimental to the purity of the formulation and limits the maximum dosage attainable.

There is thus a need for a simpler sustained-release formulation of gliclazide which does not make use of different classes of compounds for achieving the sustained-release effect. There is also a need for gliclazide formulations (or other pharmaceutical formulations) wherein a higher dosage may be attained and fewer impurities are present in the product.

### SUMMARY OF THE INVENTION

It is a first object of the invention to provide a sugar-free sustained-release gliclazide formulation comprising an intragranular part and an extragranular part, wherein the intragranular part comprises gliclazide or a salt thereof and the extragranular part comprises a release-controlling polymer, said intragranular part being binder-free and release-controlling polymer-free.

It is a second object of the invention to provide a sustained-release formulation comprising an intragranular part and an extragranular part, said formulation consisting essentially of:
- gliclazide or a salt thereof;
- an extragranular release-controlling polymer;
- a diluent; and
- a lubricant and/or glidant.

According to a particular embodiment, the formulation of the invention is according to both the first object and the second object of the invention.

According to a particular embodiment, the formulation of the invention achieves the release of 50 wt% of the gliclazide between 4 and 6 hours after immersion in phosphate buffer at pH 7.4 and which preferably achieves the release of 90 wt% of the gliclazide between 10 and 15 hours after immersion in phosphate buffer at pH 7.4.

According to a particular embodiment, the extragranular release-controlling polymer is a cellulose-derived polymer, preferably hydroxypropylmethylcellulose.

According to a particular embodiment, the formulation of the invention comprises dicalcium phosphate dihydrate as a diluent.

According to a particular embodiment, the extragranular release-controlling polymer is a mixture of
(a) hydroxypropylmethylcellulose having a viscosity of approximately 4000 cPs; and
(b) hydroxypropylmethylcellulose having a viscosity of approximately 100 cPs;
preferably wherein the weight ratio of (a) to (b) is comprised between 0.01 and 1.0, more preferably between 0.01 and 0.1, more advantageously between 0.04 and 0.08. Ideally, this ratio is approximately equal to 0.06.

It is a third object of the invention to provide a sugar-free sustained-release gliclazide formulation comprising an intragranular part and an extragranular part, said formulation consisting essentially of:
- from 10 to 30 wt%, preferably from 15 to 24 wt%, more preferably from 18 to 19.5 wt% gliclazide;
- from 10 to 40 wt%, preferably from 15 to 30 wt%, more preferably from 18 to 24 wt% release-controlling polymer;
- from 45 to 75 wt%, preferably from 50 to 69 wt%, more preferably from 57 to 63 wt% diluent;
- from 0.1 to 1 wt%, preferably from 0.3 to 0.8 wt%, more preferably from 0.4 to 0.7 wt% lubricant; and
- from 0.05 to 1 wt%, preferably from 0.1 to 0.5 wt%, more preferably from 0.15 to 0.35 wt% glidant.

According to a particular embodiment, the formulation of the invention is according to both the first object and the third object of the invention; or the formulation of the invention is according to both the second object and the third object of the invention.

According to a particular embodiment, the formulation of the invention is for treating diabetes.

According to a particular embodiment, the formulation of the invention is formulated as a tablet.

It is yet another object of the present invention to provide a process for the preparation of a sustained-release formulation, the process comprising the steps of:
a) mixing gliclazide or a salt thereof with a diluent, in the absence of any sugar-based compound and in the absence of binder, and in the absence of a release-controlling polymer to obtain a dry mix;
b) forming granules from the dry mix of step a);
c) mixing the granules of step b) with a release-controlling polymer to obtain a final blend in the absence of sugar; and
d) compressing the final blend of step c) into tablets.

According to a particular embodiment, step b) is performed by wet granulating the dry mix of step a), followed by drying.

According to a particular embodiment, step b) is performed by compressing the dry mix of step a) into slugs, followed by milling the slugs into granules.

According to a particular embodiment, a lubricant may be admixed with the gliclazide and diluent at step a).

According to a particular embodiment, a glidant and/or a lubricant are admixed with the granules and the release-controlling polymer at step c).

According to a particular embodiment of the process of the invention, the sustained-release formulation is as described above.

It is yet another object of the invention to provide a sugar-free and binder-free and release-controlling polymer-free granulate, containing an active pharmaceutical ingredient, for a sustained release formulation.

It is yet another object of the invention to provide a sugar-free sustained-release formulation comprising an intragranular part and an extragranular part, wherein the intragranular part comprises an active pharmaceutical ingredient or a salt thereof, said intragranular part being binder-free and release-controlling polymer-free.

According to a particular embodiment, the active pharmaceutical ingredient is a water-insoluble drug, preferably a sulphonyl urea anti-diabetic drug.

The present invention enables to overcome the inconveniences of the prior art. In particular the invention provides a simple sustained-release formulation of gliclazide which makes use of only one class of compounds for achieving the sustained-release effect.

This is obtained by designing a gliclazide formulation wherein the sustained-release effect is achieved only through extragranular release-controlling polymers.

Reducing the number of ingredients in the intragranular part of the formulation reduces the chance of an interaction and hence results in fewer impurities in the final product. Additionally, a binder-free and release-controlling polymer free granulate is beneficial since formulations with higher dosage may be obtained.

According to particular embodiments, the invention also has one or more of the following advantageous features:
- The formulation of the invention does not comprise any binding compound in the intragranular part of the tablet. Advantageously, more than 99%, preferably at least 99.5%, of the intragranular part of the formulation is composed of gliclazide and a diluent, while more than 95%, preferably more than 96%, of the extragranular part of the formulation is composed of one or more release-controlling polymers.
- The formulation of the invention achieves a similar or even better sustained-release effect when compared to prior art formulations, in particular with the formulations disclosed in WO 00/18373 and/or in WO 2006/123213 and/or in WO 2008/062470.
- The formulation of the invention achieves a similar or even better independence of the release profile to pH variations when compared to prior art formulations, in particular with the formulations disclosed in WO 00/18373 and/or in WO 2006/123213 and/or in WO 2008/062470.

### BRIEF DESCRIPTION OF THE DRAWINGS

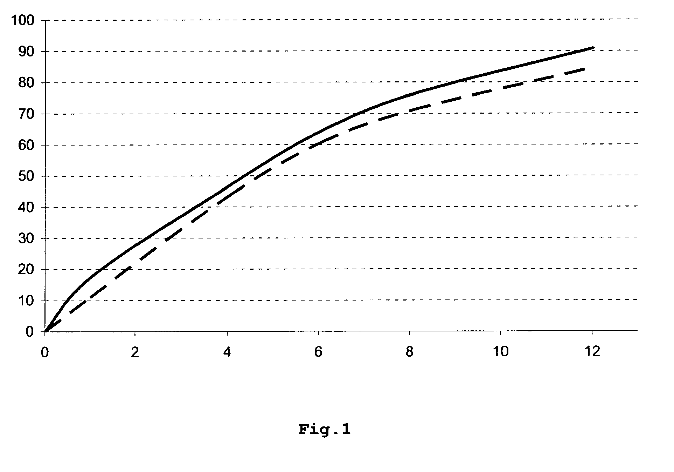

**Figure 1** is a graph showing the dissolution profiles of: the product of example 1 (full line plot); and the product of example 2 (dotted line plot). The time in hours is indicated on the X-axis and the mean cumulative weight percentage released is indicated on the Y-axis.

### DESCRIPTION OF EMBODIMENTS

The invention will now be described in more detail without limitation in the following description.

### Sustained-release gliclazide composition (or formulation)

As used herein, the phrase "sustained release" generally refers to the release of the active ingredient over an extended period of time leading to relatively lower peak plasma concentrations and a prolonged bioavailability as compared to "immediate release" formulations of the same active ingredient.

More precisely, the phrase "sustained release" refers to the release of the drug from the pharmaceutical composition over a period substantially longer than 6 hours, such as about 10 hours or longer.

Generally, the sustained release of drug from the inventive compositions occurs at such a rate that blood (for example, plasma) concentration in a patient to whom the pharmaceutical composition is administered exhibits reduced variations when compared to blood concentration in a patient to whom an immediate-release formulation is administered.

The gliclazide composition according to the invention comprises gliclazide or a salt of gliclazide as well as a cellulose-derived polymer.

The salt of gliclazide can be any pharmaceutically acceptable (crystalline or amorphous) acid or preferably basic addition salt of gliclazide such as sodium, potassium, tromethamine, lysinate salts of gliclazide.

Pharmaceutically acceptable solvates and hydrates of gliclazide are also covered by the generic term gliclazide as used herein.

The extra-granular release-controlling polymer is preferably a cellulose-derived polymer. The cellulose-derived polymer may be one or more of hydroxypropylmethylcellulose, hydroxyethylcellulose, hydroxypropylcellulose, carboxymethylcellulose, sodium carboxymethylcellulose and mixtures thereof. The one or more controlled release polymers may be present at a concentration of from 10 to 40 wt%, preferably from 15 to 30 wt%, more preferably from 18 to 24 wt%, based on the weight of the formulation. For example the cellulose-derived polymer may be hydroxypropylmethylcellulose or a mixture of hydroxypropylmethylcelluloses of different viscosities. Hydroxypropylmethylcellulose with a viscosity of 100 cPs and hydroxypropylmethylcellulose with a viscosity of 4000 cPs may be used. Advantageously, the weight ratio of hydroxypropylmethylcellulose with a viscosity of 4000 cPs to hydroxypropylmethylcellulose with a viscosity of 100 cPs is comprised between 0.01 and 1.0, more preferably between 0.01 and 0.1, more advantageously between 0.04 and 0.08.

The composition preferably contains a diluent. The diluent is a non-sugar based compound; preferably, the diluent is dicalcium hydrogen phosphate, either in an anhydrous or hydrated form, such as the dihydrate. The dicalcium hydrogen phosphate may be used as normal grade or direct compression grade. In a preferred embodiment of the present invention, direct compression grade dicalcium hydrogen phosphate dihydrate is used. Examples of other diluents that can be used include calcium sulphate, calcium triphosphate, calcium carbonate, magnesium carbonate, magnesium oxide, magnesium trisilicate, talcum. The diluent may be present at a concentration of from 45 to 75 wt%, preferably from 50 to 69 wt%, more preferably from 57 to 63 wt%, based on the weight of the formulation.

The composition preferably contains a lubricant, for instance stearic acid or its salts, such as calcium stearate, zinc stearate, more preferably magnesium stearate. Other possible lubricants include talc, glyceryl behenate, solid grades of polyethylene glycol, glyceryl palmitostearate, hydrogenated vegetable oil and mixtures of the former ingredients. The lubricant may be present at a concentration of from 0.1 to 1 wt%, preferably from 0.3 to 0.8 wt%, more preferably from 0.4 to 0.7 wt%, based on the weight of the formulation.

The composition preferably contains a glidant, more preferably colloidal silicon dioxide (anhydrous colloidal silica). The glidant may be present at a concentration of from 0.05 to 1 wt%, preferably from 0.1 to 0.5 wt%, more preferably from 0.15 to 0.35 wt%, based on the weight of the formulation.

The sustained-release formulation as described herein may be in the form of a tablet, capsule or granules. Preferably, the sustained-release formulation is a tablet. At any rate, the formulation comprises granules. The formulation thus comprises an intragranular part and an extragranular part.

By "intragranular" is meant the part of the formulation which constitutes the granules. "Intragranular" is synonymous with "granular".

By "extragranular" is meant the part of the formulation which is not comprised in the granules. "Extragranular" is synonymous with "intergranular". Generally speaking, the extragranular part is added to the granules to form tablets.

The intragranular part preferably comprises gliclazide, the diluent and optionally part of the lubricant; and the extragranular part preferably comprises the cellulose-derived polymer and optionally the glidant and/or the lubricant or part of the lubricant.

The intragranular part does not substantially contain any binder or release-controlling polymer or sugar-based compound, and only in the extra-granular part is a release-controlling polymer substantially present in the composition.

The sugar-based compounds substantially excluded from the composition are all monosaccharides, disaccharide, trisaccharides and polysaccharides, or mono, di- or trisaccharide/polysaccharide derivatives. In particular, glucose, lactose, glucose syrup, maltodextrin and starch are excluded, as well as all polyols and sugar alcohols.

The binders substantially excluded from the intragranular composition are all materials known by the skilled in the art of pharmaceutical formulation as having the property of holding together the structure of dosage forms, i.e. of binding together all the other ingredients after sufficient compression forces have been applied and of providing the integrity of the dosage forms. The term "binders" covers both water-soluble and insoluble binders, including those soluble binders which can also serve as water soluble drug release modifying agents. Alternatively, water soluble salts or excipients acting as weak binders and which may be used to modulate the drug release profile are comprised within the term "binders".

In particular, polyvinylpyrrolidone, pregelatinised starches, cellulose, carbomers, sucrose, lactose, dextrose, sorbitol, mannitol and gums are excluded.

The release-controlling polymer substantially excluded from the intragranular part of the composition are all materials known by the skilled in the art of pharmaceutical formulation as having the property of controlling the release of drug from the tablet when it is placed in a suitable aqueous medium. The term "release-controlling polymer" covers a wide range of polymers of synthetic, semi-synthetic and natural origin. The polymers could be water swellable or non-swellable in nature. These include cellulose or cellulose-derived polymers, such as, carboxymethylcellulose and salts thereof, methylcellulose, hydroxyethylcellulose, hydroxypropylcellulose, hydroxypropylmethylcellulose, hydroxyethylmethylcellulose, polyethylene oxides, natural gums, such as, but not limited to xanthan gum and guar gum, carrageenans, agar, alginic acid and its salts, carbomers, ethylcellulose, methacrylic acid polymers, polyacrylic acid polymers and copolymers of acrylic acid and methacrylic acid and derivatives thereof, gelatin, hydroxypropyl starch and other suitable starch derivatives, pectinic acid, pectin and derivatives thereof.

In particular, cellulose or cellulose-derived polymers, such as, hydroxypropyl methylcellulose, methylcellulose, hydroxyethyl cellulose, hydroxypropylcellulose, sodium carboxymethylcellulose, hydroxyethylmethylcellulose either used individually or in any combination are excluded.

"Free of" in the context of the invention means less than 0.5%, preferably less than 0.2%, less than 0.1%, more preferably less than 0.01% or even below the level of detection by conventional techniques.

In a more advantageous embodiment no binder or sugar-based compound or release-controlling polymer is present in the intragranular part, save contamination. Besides, the extragranular part is also preferably sugar-free and/or binder-free but preferably contains a release-controlling polymer.

"Sugar-free" refers to the sugar-based compounds listed above.

"Binder-free" refers to the binders listed above.

"Release-controlling polymer-free" refers to the release-controlling polymers listed above.

"Consisting essentially of" is intended to exclude those compositions comprising a binder (especially more than 0.5%) and/or a sugar-based compound (especially more than 0.5%), and/or a release-controlling polymer in the intragranular part (especially more than 0.5%). "Consisting essentially of" is especially intended to cover those compositions having less than 0.5%, preferably less than 0.2%, more preferably less than 0.1%, ideally less than 0.01%, of compounds other than those cited. According to an embodiment, "consisting essentially of" means "consisting of" and thus covers those compositions which do not contain any compounds other than those cited.

It is yet another aspect of the present invention to provide a sugar-free and binder-free and release-controlling polymer-free granulate for use in sustained release formulations. Said sugar-free and binder-free and release-controlling polymer-free granulate may comprise any suitable active pharmaceutical ingredient. Preferably, the active pharmaceutical ingredient falls within the category of water insoluble drugs. Suitably, the active pharmaceutical ingredient is not prone to degradation in the presence of water or heat. For example, anti-diabetic drugs that fall in the category of sulphonyl ureas may be used. Examples of such sulphonyl ureas are glipizide, glibenclamide, glimepiride, gliquidone, glycopyramide and gliclazide.

Suitably, the sugar-free and binder-free and release-controlling polymer-free granulate of the present invention can be used as such or in a conventional release formulation such as a direct release formulation.

In other terms, the present description may also be read with the expression "active pharmaceutical ingredient" replacing the word "gliclazide" in all occurrences.

### Process for preparing the sustained-release gliclazide composition

The formulation/composition may be obtained by a direct compression method, or alternatively by a wet granulation method or dry granulation/slugging method.

A process for the preparation of a gliclazide matrix tablet by wet granulation may include the following steps:
- Mixing the gliclazide together with the diluent, followed by wetting of that mixture with purified water. The resulting wet mass is then granulated, dried and subsequently classified to obtain a granulate having physical characteristics that enable good filling of the moulds of a rapid-compression machine. The drying step may suitably be performed using a drying cabinet or a fluid bed dryer or using infra-red radiation or microwaves, either with or without the aid of vacuum.
- Mixing the granulate thus obtained with the release-controlling polymer.
- Adding the lubricant and glidant.
- Compressing the lubricated mixture thus obtained using a rotary compression machine to obtain tablets.

A process for the preparation of a gliclazide matrix tablet by dry granulation may include the following steps:
- Mixing the gliclazide and diluent, optionally together with part of the lubricant.
- Compressing the dry mix into slugs using a suitable tablet compression machine or a compactor.
- Milling the slugs using a suitable mill.
- Blending the granules with the release-controlling polymer, the glidant and part or all of the lubricant.
- Compressing the blend to tablets.

As an alternative to dry granulation, the blended composition comprising all the ingredients may be compressed directly into the final pharmaceutical dosage form using a conventional direct compression technique well known in the art. It produces a uniform tablet without granules.

A wet granulation process and a dry granulation/slugging process are respectively illustrated in examples 1 and 2 below.

### EXAMPLES

The following examples illustrate the invention without limiting it.

### Example 1

Sustained-release gliclazide tablets according to the invention were prepared with the following ingredients:

| **Ingredients** | **mg per tablet** | **kg (for 400,000 tablets)** |
|---|---|---|
| **Intra-granular** | | |
| Gliclazide Ph.Eur. | 30.00 | 12.000 |
| Dicalcium phosphate | 92.88 | 37.152 |
| dihydrate Ph.Eur. | | |

| Extra-granular | | |
|---|---|---|
| HPMC 4000 cPs | 2.00 | 0.800 |
| HPMC 100 cPs | 34.00 | 13.600 |
| Colloidal silicon dioxide | 0.32 | 0.128 |
| Magnesium stearate | 0.80 | 0.320 |

The tablets were prepared by a wet granulation process as follows, based on a 400,000 tablet batch size:
- The gliclazide and dicalcium phosphate were mixed in a Rapid Mixer Granulator.
- Purified water was added while mixing. The granules were unloaded and transferred to a drying cabinet for drying.
- The dried granules were loaded into a drum / double cone mixer, together with hydroxypropylmethylcellulose of a viscosity of 4,000 cPs (Methocel K4M CR Premium by Dow), hydroxypropylmethylcellulose of a viscosity of 100 cPs (Methocel K100 LV CR Premium by Dow) and colloidal silicon dioxide.
- The magnesium stearate was added to the blend in the mixer and mixing was further carried out.
- The blend was then compressed to tablets using a Fette tablet compression machine.

### Example 2

Sustained-release gliclazide tablets according to the invention were prepared with the following ingredients:

| **Ingredients** | **mg per tablet** | **kg (for 400,000 tablets)** |
|---|---|---|
| **Intra-granular** | | |
| Gliclazide Ph.Eur. | 30.00 | 12.000 |
| Dicalcium phosphate | 100.08 | 40.030 |
| dihydrate Ph.Eur. | | |

| **Extra-granular** | | |
|---|---|---|
| HPMC 4000 cPs | 2.00 | 0.800 |
| HPMC 100 cPs | 26.00 | 10.400 |
| Colloidal silicon dioxide | 0.32 | 0.128 |
| Magnesium stearate | 1.60 | 0.640 |

The tablets were prepared by a dry granulation process as follows, based on a 400,000 tablet batch size:
- The gliclazide and dicalcium phosphate were mixed in a double cone/drum mixer.
- The dry mix was compressed into slugs and subsequently milled to form granules.
- The obtained gliclazide mixture was blended with hydroxypropylmethylcellulose of a viscosity of 4,000 cPs (Methocel K4M CR Premium by Dow), hydroxypropylmethylcellulose of a viscosity of 100 cPs (Methocel K100 LV CR Premium by Dow) and colloidal silicon dioxide using a drum / double cone mixer.
- Magnesium stearate was added to the blend in the mixer and mixing was further carried out.
- The blend was then compressed to tablets.

### Example 3

The dissolution profile of the compositions of example 1 and example 2 above were determined, as well as the dissolution profile of commercial product Diamicron® 30 mg MR by Servier, which contains 30 mg of gliclazide, in association with hypromellose, maltodextrin, calcium hydrogen phosphate dihydrate, magnesium stearate and anhydrous colloidal silica.

The following dissolution conditions were used for all experiments:
Dissolution apparatus type: USP 24 Type I (Basket)
Paddle speed: 100 rpm
Dissolution medium: phosphate buffer, pH 7.4
Dissolution volume: 900 ml
Sampling quantity: 10 ml
Temperature of dissolution medium: 37° ± 0.5°C

The dissolution profiles obtained may be seen on **Fig.1****.** The numerical results are presented in the table below (the figures correspond to the mean cumulative percentage of gliclazide released at a given time).

| Time (hours) | Example 1 | Example 2 |
|---|---|---|
| 0 | 0 | 0 |
| 1 | 12.0 | 16.0 |
| 4 | 41.3 | 49.1 |
| 8 | 72.0 | 73.4 |
| 12 | 85.6 | 82.9 |

The table and the graph show that the dissolution profiles of the products according to the invention achieve an excellent controlled release.

In the above description, any composition or process or use embodied for gliclazide can be embodied with any other active pharmaceutical ingredient.

## Claims

1. A sugar-free sustained-release gliclazide formulation comprising an intragranular part and an extragranular part, wherein the intragranular part comprises gliclazide or a salt thereof and the extragranular part comprises a release-controlling polymer, said intragranular part being binder-free and release-controlling polymer-free.

2. A sustained-release formulation comprising an intragranular part and an extragranular part, said formulation consisting essentially of:
- gliclazide or a salt thereof;
- a diluent;
- an extra-granular release-controlling polymer;
- a lubricant and/or glidant.

3. The formulation of claim 1 or 2, which achieves the release of 50 wt% of the gliclazide between 4 and 6 hours after immersion in phosphate buffer at pH 7.4 and which preferably achieves the release of 90 wt% of the gliclazide between 10 and 15 hours after immersion in phosphate buffer at pH 7.4.

4. The formulation of one of claims 1 to 3, wherein the extra-granular release-controlling polymer is a cellulose-derived polymer, and preferably hydroxypropylmethylcellulose.

5. The formulation of one of claims 1 to 4, comprising dicalcium phosphate as a diluent.

6. The formulation of one of claims 1 to 5, wherein the extra-granular release-controlling polymer is a mixture of
(a) hydroxypropylmethylcellulose having a viscosity of approximately 4000 cPs; and
(b) hydroxypropylmethylcellulose having a viscosity of approximately 100 cPs;
preferably wherein the weight ratio of (a) to (b) is comprised between 0.01 and 1.0, more preferably between 0.01 and 0.1, more advantageously between 0.04 and 0.08.

7. A sugar-free sustained-release gliclazide formulation comprising an intragranular part and an extragranular part, said formulation consisting essentially of:
- from 10 to 30 wt%, preferably from 15 to 24 wt%, more preferably from 18 to 19.5 wt% gliclazide;
- from 10 to 40 wt%, preferably from 15 to 30wt%, more preferably from 18 to 24 wt% extragranular release-controlling polymer;
- from 45 to 75 wt%, preferably from 50 to 69 wt%, more preferably from 57 to 63 wt% diluent;
- from 0.1 to 1 wt%, preferably from 0.3 to 0.8 wt%, more preferably from 0.4 to 0.7 wt% lubricant; and
- from 0.05 to 1 wt%, preferably from 0.1 to 0.5 wt%, more preferably from 0.15 to 0.35 wt% glidant.

8. The formulation of one of claims 1 to 7, for treating diabetes.

9. The formulation of one of claims 1 to 8, as a tablet.

10. A process for the preparation of a sustained-release formulation, the process comprising the steps of :
a) mixing gliclazide or a salt thereof with a diluent, in the absence of any sugar-based compound and in the absence of binder and in the absence of a release-controlling polymer, to obtain a dry mix;
b) forming granules from the dry mix of step a);
c) mixing the granules of step b) with a release-controlling polymer to obtain a final blend in the absence of sugar; and
d) compressing the final blend of step c) into tablets.

11. The process of claim 10, wherein step b) is performed by wet granulating the dry mix of step a), followed by drying.

12. The process of claim 10, wherein step b) is performed by compressing the dry mix of step a) into slugs, followed by milling the slugs into granules.

13. The process of one of claims 10 to 12, wherein a lubricant is admixed with the gliclazide and diluent at step a).

14. The process of one of claims 10 to 13, wherein a glidant and/or a lubricant are admixed with the granules and the release-controlling polymer at step c).

15. The process of one of claims 10 to 14, wherein the sustained-release formulation is according to any one of claims 1 to 9.

16. A sugar-free and binder-free and release-controlling polymer-free granulate, containing an active pharmaceutical ingredient, for a sustained release formulation.

17. A sugar-free sustained-release formulation comprising an intragranular part and an extragranular part, wherein the intragranular part comprises an active pharmaceutical ingredient or a salt thereof, said intragranular part being binder-free and release-controlling polymer-free.

18. The formulation according to claim 16, wherein the active pharmaceutical ingredient is a water-insoluble drug, preferably a sulphonyl urea anti-diabetic drug.
